# EUROPÄISCHE PATENTSCHRIFT

(11) **EP 1 222 191 B1**
(45) Veröffentlichungstag und Bekanntmachung des Hinweises auf die Patenterteilung: **16.11.2005**
(21) Anmeldenummer: 00966022.6
(22) Anmeldetag: 15.09.2000
(51) Int. Cl.: C07D 487/06, A61K 31/5517, A61K 31/519, A61P 25/00, A61P 35/00, C07D 243/14

(54) **BENZODIAZEPIN-DERIVATE, DEREN HERSTELLUNG UND ANWENDUNG**
BENZODIAZEPIN DERIVATIVES, THE PRODUCTION AND USE THEREOF
DERIVES DE BENZODIAZEPINE, PREPARATION ET UTILISATION DESDITS DERIVES

(30) Priorität: 28.09.1999 DE 19946289
(43) Veröffentlichungstag der Anmeldung: 17.07.2002
(73) Patentinhaber: Abbott GmbH & Co. KG, 65205 Wiesbaden (DE)
(72) Erfinder: LUBISCH, Wilfried, 69115 Heidelberg (DE); KOCK, Michael, 67105 Schifferstadt (DE); HÖGER, Thomas, 68535 Edingen-Neckarhausen (DE); GRANDEL, Roland, 69221 Dossenheim (DE); MÜLLER, Reinhold, 67105 Schifferstadt (DE); SCHULT, Sabine, 67346 Speyer (DE)
(74) Vertreter: Grünecker, Kinkeldey, Stockmair & Schwanhäusser Anwaltssozietät
(86) Internationale Anmeldenummer: PCT/EP2000/009023
(87) Internationale Veröffentlichungsnummer: WO 2001/023386

(56) Entgegenhaltungen:
- EP-A- 0 384 522
- WO-A-01/16136
- WO-A-97/04771
- GENESTE, P. ET AL: "Studies on the imidazo[4,5,1-jk][1,4]benzodiazepine and imidazo[1,5,4-ef][1,5]benzodiazepine series" EUR. J. MED. CHEM. - CHIM. THER. (1978), 13(1), 53-9 , XP002169307

## Beschreibung

Die vorliegende Erfindung betrifft neuartige Benzodiazepin-Derivate, ihre Herstellung und die Verwendung als Inhibitoren des Enzyms Poly(ADP-ribose)polymerase oder PARP (EC 2.4.2.30) zur Herstellung von Arzneimitteln.

Poly(ADP-ribose)polymerase (PARP) bzw. wie es auch genannt wird Poly(ADP-ribose)synthase (PARS) stellt ein regulatorisches Enzym dar, das in Zellkernen gefunden wird (K. Ikai et al., J. Histochem. Cytochem. 1983, 31, 1261-1264). Man nimmt an, daß PARP eine Rolle bei der Reparatur von DNA-Brüchen spielt (M.S. Satoh et al., Nature 1992, 356, 356-358). Schädigungen oder Brüche der DNA-Stränge aktivieren das Enzym PARP, das, wenn es aktiviert ist, die Übertragung von ADP-Ribose aus NAD katalysiert (S. Shaw, Adv. Radiat. Biol., 1984, 11, 1-69). Dabei wird Nikotinamid aus NAD freigesetzt. Nikotinamid wird unter Verbrauch des Energieträgers ATP von anderen Enzymen wieder in NAD umgewandelt. Eine Überaktivierung von PARP hätte dementsprechend einen unphysiologisch hohen Verbrauch von ATP zur Folge und dies führt im Extremfall zu Zellschädigungen und Zelltod.

Es ist bekannt, daß Radikale wie Superoxid-Anion, NO und Wasserstoffperoxid in Zellen zu DNA-Schädigungen führen können und damit PARP aktivieren. Die Bildung von großen Mengen an Radikalen wird bei einer Reihe von pathophysiologischen Zuständen beobachtet und man geht davon aus, daß diese Anhäufung von Radikalen zu den beobachteten Zell- bzw Organschäden führt oder beiträgt. Dazu zählt zum Beispiel ischämische Zustände von Organen wie im Schlaganfall, Herzinfarkt (C. Thiemermann et al., Proc. Natl. Acad. Sci. USA, 1997, 94, 679-683) oder Ischämie der Nieren, aber auch Reperfusionsschäden wie sie zum Beispiel nach der Lyse von Herzinfarkt auftreten (s. oben: C. Thiemermann et al.). Die Hemmung von dem Enzym PARP könnte demzufolge ein Mittel sein, um diese Schäden zum mindestens zum Teil zu verhindern oder abzumildern. PARP-Inhibitoren könnten somit ein neues Therapieprinzip zur Behandlung von eine Reihe von Krankheiten darstellen.

Das Enzym PARP beeinflußt die Reparatur von DNA-Schäden und könnte somit auch in der Therapie von Krebs-Erkrankungen eine Rolle spielen, da in Kombination mit cytostatisch wirksamen Stoffen ein höheres Wirkpotential gegenüber Tumorgewebe beobachtet wurde (G. Chen et al. Cancer Chemo. Pharmacol. 1988, 22, 303).

Nicht limitierende Beispiele für Tumoren sind Leukämie, Glioblastome, Lymphome, Melanome, Mamma- und Zervikalkarzinome.

Zudem wurde gefunden, daß PARP-Inhibitoren immunosuppressive Wirkung zeigen können (D. Weltin et al. *Int. J. Immunopharmacol*. 1995, 17, 265-271).

Es wurde ebenfalls entdeckt, daß PARP bei immunologischen Erkrankungen bzw. Krankheiten, in denen das Immunsystem eine wichtige Rolle spielt, wie zum Beispiel rheumatoide Arthritis und septischer Schock, involviert ist, und daß PARP-Inhibitoren einen günstigen Effekt auf den Krankheitsverlauf zeigen können (H. Kröger et al. *Inflammation* 1996, 20, 203-215; W.Ehrlich et al. *Rheumatol. Int*. 1995, 15, 171-172; C. Szabo et al., *Proc. Natl. Acad. Sci. USA* 1998, 95, 3867-3872; S. Cuzzocrea et al. *Eur. J. Pharmacol.* 1998, 342, 67-76).

Unter PARP im Sinne dieser Erfindung werden auch Isoenzyme des oben beschriebenen PARP-Enzyms verstanden.

Weiterhin zeigte der PARP-Inhibitor 3-Aminobenzamid protektive Effekte in einem Model für den Kreislaufschock (S. Cuzzocrea et al., *Br. J. Pharmacol.* 1997, 121, 1065-1074).
Ebenfalls gibt es experimentelle Hinweise, dass Inhibitoren des Enzymes PARP als Mittel zur Behandlung von Diabetes mellitus nützlich sein könnten (V. Burkart et al. *Nature Med*. 1999, 5, 314-319).

Benzodiazepine und Benzodiazepinone und deren Derivate stellen eine chemische Klasse dar, die vielfach in der organischen Synthese Verwendung fanden. Derivate dieser Verbindungen, die zudem einen Imidazo-Ring anelliert tragen, das heißt Imidazobenzodiazepinone, sind jedoch kaum beschrieben worden. Aminodibenzodiazepinone wurden in P.V. Khadikar et al. J. Heterocycl. Chem. 1998, 35, 675 hergestellt. So wurden in Geneste et al. Eur. J. Chem. Chim. Ther. 1978, 13, 53 einfache Derivate hergestellt, die am Benzo-Ring Reste wie Chlor oder Nitro und am Imidazo-Ring eine Methyl-Gruppe tragen. In M.J. Kukla et al., J. Med. Chem. 1991, 34, 3187, wurde ein Dihydro-imidazo-benzodiazepinon als Zwischenverbindung für Wirkstoffe, die anti-HIV-Wirkung zeigen sollen, hergestellt.

In der WO 97/04771 werden Benzimidazol-4-carboxamide beschrieben, sowie deren Verwendung für die Herstellung von Medikamenten, die eine hemmende Wirkung auf das Enzym Poly(ADP-ribose)polymerase (PARP) ausüben.

Die hier erfindungsgemäßen Verbindungen der allgemeinen Formel I sind bisher nicht beschrieben worden und sind demnach neu.

Es wurde weiterhin überraschenderweise gefunden, daß Benzodiazepin-Derivate, die einen anellierten Ring tragen, gut wirksame Inhibitoren für das Enzym PARP darstellen.

In der vorliegenden Erfindung werden neue Benzodiazepin-Derivate der allgemeinen Formel I beschrieben, die potente PARP-Inhibitoren darstellen.

Gegenstand der vorliegenden Erfindung sind substituierte Benzodiazepin-Derivate der allgemeinen Formel I 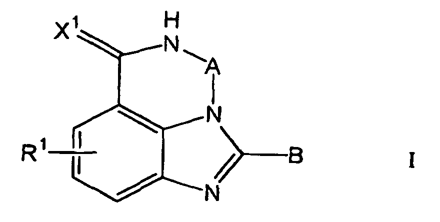 worin
- A: eine C₁-C₃-Kohlenwasserstoffkette ist, wobei jedes Kohlenstoffatom noch einen oder zwei der folgenden Substituenten tragen kann:
C₁-C₄-Alkyl, OH, O-C₁-C₄-Alkyl, COOH, COO-C₁-C₄-Alkyl und Phenyl oder ein C-Atom auch eine =O -Gruppe tragen kann und
- X¹: S, O oder NH ist und
- R¹: Wasserstoff, Chlor, Fluor, Brom, Iod, verzweigtes oder unverzweigtes C₁-C₆-Alkyl, OH, Nitro, CF₃, CN, NR¹¹R¹², NH-CO-R¹³ oder O-C₁-C₄-Alkyl ist, wobei R¹¹ und R¹² unabhängig voneinander Wasserstoff oder C₁-C₄-Alkyl bedeuten und R¹³ Wasserstoff, C₁-C₄-Alkyl, C₁-C₄-Alkyl-Phenyl oder Phenyl bedeutet, und
- B: Piperidin oder Piperazin, die noch mit einen R⁴ oder maximal 2 R⁵ substituiert sein können, bedeutet, und
- R⁴: Wasserstoff oder -(D)ₚ-(E)ₛ- (F¹)_{q}-G¹-(F²)ᵣ-(G²)-G³ bedeutet, wobei
- D: S, NR⁴³ oder O

- E: Phenyl, 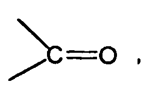 -SO₂-, -SO₂NH-, -NHCO-, -CONH-, -NHSO₂-, -NHCOCH₂X⁴-, und
- X⁴: S, O oder NH bedeutet, und
- F¹: eine geradkettige oder verzweigte gesättigte oder ungesättigte Kohlenstoffkette mit 1 bis 8 C-Atomen ist und
- F²: unabhängig von F¹ die gleiche Bedeutung wie F¹ besitzt,
- G¹: eine Bindung bedeutet oder einen ungesättigten, gesättigten oder partialungesättigten mono-, bi- oder tricyclischen Ring mit maximal 15 Kohlenstoffatomen, einen ungesättigten, gesättigten oder partial-ungesättigten mono-, bi- oder tricyclischen Ring mit maximal 14 Kohlenstoffatomen und 0 bis 5 Stickstoffatomen, 0 bis 2 Sauerstoffatomen bzw. 0 bis 2 Schwefelatomen bedeutet, die jeweils noch mit maximal 3 unterschiedlichen oder gleichen Resten R⁵ substituiert sind, und ein oder zwei Kohlenstoff- bzw. Schwefelatome auch ein oder zwei =O -Gruppen tragen können, und
G² a) NR⁴¹R⁴² oder 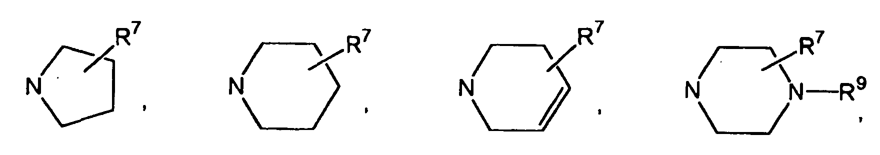 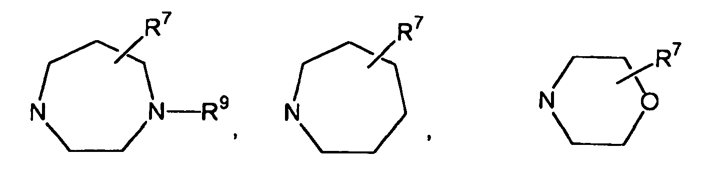 bedeutet, dann ist G³ ein Wasserstoff
oder G² b) eine Bindung ist, dann bedeutet
- G³: einen ungesättigten, gesättigten oder partial-ungesättigten mono-, bi- oder tricyclischen Ring mit maximal 15 Kohlenstoffatomen, einen ungesättigten, gesättigten oder partial-ungesättigten mono-, bi-oder tricyclischen Ring mit maximal 14 Kohlenstoffatomen und 0 bis 5 Stickstoffatomen, 0 bis 2 Sauerstoffatomen bzw. 0 bis 2 Schwefelatomen, die jeweils noch mit maximal 3 unterschiedlichen oder gleichen Resten R⁵ substituiert sind, und ein oder zwei Kohlenstoff- bzw. Schwefelatome auch ein oder zwei =O -Gruppen tragen können, oder Wasserstoff bedeutet, und
- p: 0 oder 1 bedeutet und
- s: 0 oder 1 und
- q: 0 oder 1 sein kann und
- r: 0 oder 1 sein kann und
- R⁴¹: Wasserstoff, C₁-C₆-Alkyl, wobei jedes Kohlenstoffatom noch bis zu zwei Reste R⁶ tragen kann, Phenyl, der noch maximal zwei Reste R⁶ tragen kann, oder (CH₂)ₜ-K ist, und
- R⁴²: Wasserstoff, C₁-C₆-Alkyl, CO-R⁸, CO₂-R⁸, SO₂NH₂, SO₂-R⁸, -(C=NH)-R⁸ oder -(C=NH)-NHR⁸ ist, und
- R⁴³: Wasserstoff oder C₁-C₄-Alkyl, und
- t: 1, 2, 3 oder 4 ist und
- K: NR¹¹R¹², NR¹¹-C₁-C₄-Alkyl-Phenyl, Pyrrolidin, Piperidin, 1,2,5,6-Tetrahydropyridin, Morpholin, Homopiperidin, Piperazin, das noch mit einem Alkyl-Rest C₁-C₆-Alkyl substituiert sein kann, oder Homopiperazin, das noch mit einem Alkyl-Rest C₁-C₆-Alkyl substituiert sein kann, und
- R⁵: Wasserstoff, Chlor, Fluor, Brom, lod, OH, Nitro, CF₃, CN, NR¹¹R¹², NH-CO-R¹³, C₁-C₄-Alkyl-CO-NH-R¹³, COR⁸, C₀-C₄-Alkyl-O-CO-R¹³, C₁-C₄-Alkyl-Phenyl, Phenyl, CO₂-C₁-C₄-Alkyl, verzweigtes oder unverzweigtes C₁-C₆-Alkyl, O-C₁-C₄-Alkyl, S-C₁-C₄-Alkyl ist, wobei jedes C-Atom der Alkylketten bis zu zwei Reste R⁶ tragen kann und die Alkylketten auch ungesättigt sein können, und
- R⁶: Wasserstoff, Chlor, Fluor, Brom, lod, verzweigtes oder unverzweigtes C₁-C₆-Alkyl, OH, Nitro, CF₃, CN, NR¹¹R¹², NH-CO-R¹³, O-C₁-C₄-Alkyl ist, und
- R⁷: Wasserstoff, C₁-C₆-Alkyl, Phenyl, wobei der Ring noch mit bis zu zwei Resten R⁷¹ substituiert sein kann, ein Amin NR¹¹R¹² oder ein zyklisches gesättigtes Amin mit 3 bis 7 Gliedern, das noch mit einem Alkyl-Rest C₁-C₆-Alkyl substituiert sein kann, oder Homopiperazin, das noch mit einem Alkyl-Rest C₁-C₆-Alkyl substituiert sein kann, und
wobei die Reste R¹¹, R¹² und R¹³ in K, R⁵, R⁶ und R⁷ unabhängig voneinander die gleiche Bedeutung annehmen können wie R¹, und
- R⁷¹: OH, C₁-C₆-Alkyl, O-C₁-C₄-Alkyl, Chlor, Brom, Iod, Fluor, CF₃, Nitro oder NH₂ ist, und
- R⁸: C₁-C₆-Alkyl, CF₃, Phenyl oder C₁-C₄-Alkyl-Phenyl ist, wobei der Ring noch mit bis zu zwei Resten R⁸¹ substituiert sein kann, und
- R⁸¹: OH, C₁-C₆-Alkyl, O-C₁-C₄-Alkyl, Chlor, Brom, Iod, Fluor, CF₃, Nitro oder NH₂ ist, und
- R⁹: Wasserstoff, C₁-C₆-Alkyl, C₁-C₄-Alkyl-Phenyl, CO₂-C₁-C₄-Alkyl-Phenyl, CO₂-C₁-C₄-Alkyl, SO₂-Phenyl, COR⁸ oder Phenyl ist, wobei die Phenyl-Ringe noch mit bis zu zwei Resten R⁹¹ substituiert sein können, und
- R⁹¹: OH, C₁-C₆-Alkyl, O-C₁-C₄-Alkyl, Chlor, Brom, Iod, Fluor, CF₃, Nitro oder NH₂ ist,
sowie ihre tautomeren Formen, möglichen enantiomeren und diastereomeren Formen, und deren Prodrugs.

Bevorzugt sind die Verbindungen der Formel I, wobei
- A: eine C₂-Kohlenwasserstoff-Kette ist, die substituiert sein kann, und
- X¹: O darstellt und
- R¹: Wasserstoff ist.

Ganz besonders bevorzugt sind Verbindungen der Formel I, wobei
- R⁴: Wasserstoff oder D_{0,1}-F¹_{0,1}-G²-G³ mit G³ gleich Wasserstoff bedeutet und
- D: O und NR⁴³, wobei R⁴³ Wasserstoff und C₁-C₃-Alkyl und
- F¹: C₂-C₄-Alkyl.
bedeutet.

Weiterhin sind die folgenden Verbindungen, ausgewählt aus der Gruppe bestehend aus 2-(6-Nitro-1,3-benzodioxol-5yl)-5,6-dihydroimidazo[4,5,1-*jk*][1,4]benzodiazepin-7(4H)-on, 2-(2,3-Dihydro-1,3-benzodioxin-6-yl)-5,6-dihydroimidazo[4,5,1-*jk*][1,4]benzo-diazepin-7(4H)-on, 2-(1,3-Benzodioxol-5-yl)-5,6-dihydroimidazo[4,5,1-*jk*][1,4] benzodiazepin-7(4H)-on, 2-(2,5-Dimethoxytetrahydro-3-furanyl)-5,6-dihydroimidazo[4,5,1-*jk*][1,4]benzodiazepin-7(4H)-on, 2-(2,3-Dihydro-1-benzofuran-5-yl)-5,6-dihydroimidazo[4,5,1-*jk*][1,4]benzodiazepin-7(4H)-on, 2-(6-Chlor-1,3-benzodioxol-5-yl)-5,6-dihydro-imidazo[4,5,1-*jk*][1,4]benzodiazepin-7(4H)-on sowie ihre tautomeren Formen, möglichen enantiomeren und diastereomeren Formen, und deren Prodrugs, Gegenstand der vorliegenden Erfindung.

Die Verbindungen der Formel I können als Racemate, als enantiomerenreine Verbindungen oder als Diastereomere eingesetzt werden. Werden enantiomerereine Verbindungen gewünscht, kann man diese beispielsweise dadurch erhalten, daß man mit einer geeigneten optisch aktiven Base oder Säure eine klassische Racematspaltung mit den Verbindungen der Formel I oder ihren Zwischenprodukten durchführt.

Alkylketten können jeweils verzweigt oder unverzweigt sein. Unverzweigte Alkylketten sind bevorzugt.

Gegenstand der Erfindung sind auch zu Verbindungen der Formel I mesomere oder tautomere Verbindungen.

Ein weiterer Gegenstand der Erfindung sind die physiologisch verträglichen Salze der Verbindungen I, die sich durch Umsatz von Verbindungen I mit einer geeigneten Säure oder Base erhalten lassen. Geeignete Säuren und Basen sind zum Beispiel in Fortschritte der Arzneimittelforschung, 1966, Birkhäuser Verlag, Bd. 10, S. 224-285, aufgelistet. Dazu zählen zum Beispiel Salzsäure, Citronensäure, Weinsäure, Milchsäure, Phosphorsäure, Methansulfonsäure, Essigsäure, Ameisensäure, Maleinsäure, Fumarsäure usw. bzw. Natriumhydroxid, Lithiumhydroxid, Kaliumhydroxid und Tris.

Unter Prodrugs werden solche Verbindungen verstanden, die in vivo in Verbindungen der allgemeinen Formel I metabolisiert werden. Typische Prodrugs sind Phosphate, Carbamate von Aminosäuren, Ester und andere.

Die Herstellung der erfindungsgemäßen Benzodiazepin-Derivate I kann auf verschiedenen Wegen erfolgen und wurde in den Syntheseschemata 1-3 skizziert.

Die möglichen Synthesemethoden sind im wesentlichen bereits bekannt oder orientieren sich an bekannten analogen Wegen. Syntheseschema 1

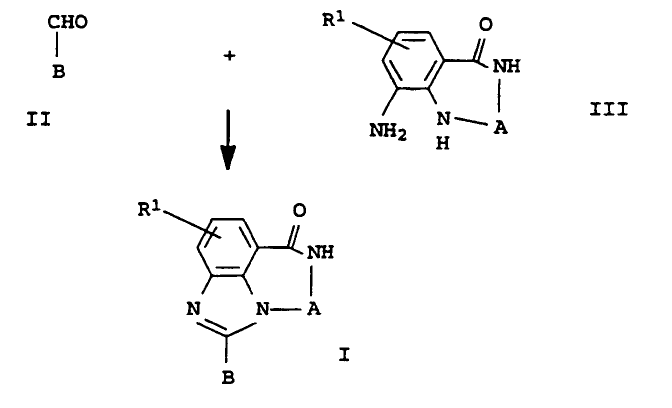

Durch Kondensation des Aldehyds II mit Diaminen III erhält man das Benzimidazol I, wobei man bevorzugt in polaren Lösungsmitteln wie Ethanol oder Dimethylformamid unter Zusatz von Säuren wie Essigsäure bei erhöhter Temperatur arbeitet, in der Regel 80-120°C. Günstig für die Reaktion ist der Zusatz von schwachen Oxidationsmittel wie z.B. Kupfer-II-Salzen, die z.B. als wäßrige Lösungen zugesetzt werden.

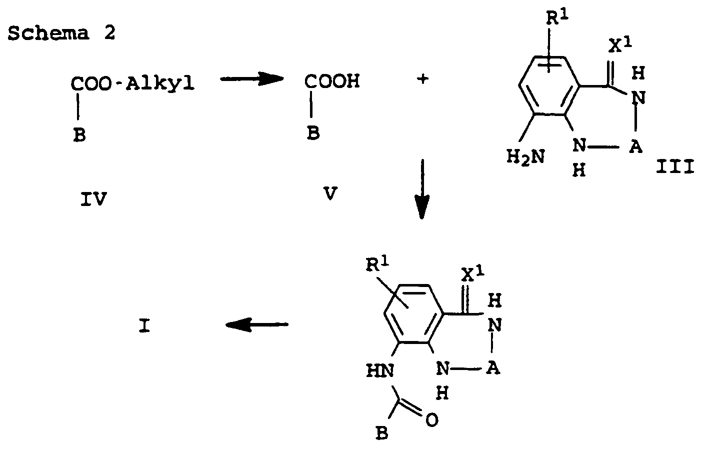

Alternativ zu den im Schema 1 gezeigten Aldehyden II kann man auch Benzoesäuren wie V (siehe Schema 2) oder Benzonitrile wie VII(siehe Schema 3) anstelle des Aldehyds einsetzen. Die Umsetzung dieser Derivate erfolgt analog zur Herstellung der substituierten Aldehyde II. Ausgehend von V erfolgt die Kondensation zu II in zwei Stufen. Zuerst wird die Benzoesäure V mit dem Anilin III in einer peptidartigen Kupplung zum Amid VI umgesetzt. Dabei arbeitet man nach üblichen Bedingungen, die zum Beispiel im Houben-Weyl, Methoden der Organischen Chemie, 4. Aufl., E5, Kap. V bzw. R.C. Larock, Comprehensive Organic Transformations, VCH Publisher, 1989, Seite 972f. aufgelistet sind. Der Ringschluß erfolgt zum Benzimidazol danach bei erhöhter Temperatur, zum Beispiel 60 bis 180°C, mit oder ohne Lösungsmitteln wie Dimethylformamid, unter Zusatz von Säuren wie Essigsäure oder direkt in Essigsäure selbst.

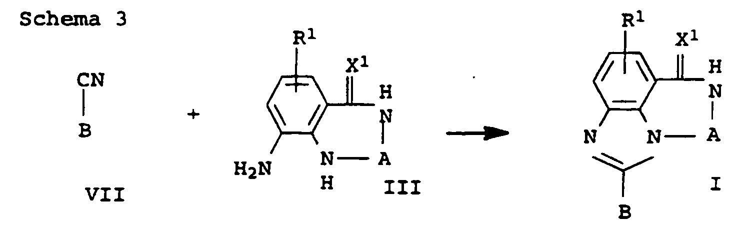

Die Reaktion des Diamins III mit einem Nitril VII erfolgt ebenfalls unter üblichen Bedingungen. Dabei kann man in Lösungsmitteln wie Dimethylformamid unter Zusatz von Säuren oder auch in Polyphosphorsäure bei erhöhter Temperatur wie 60 bis 200°C arbeiten. Allerdings kann man auch die üblichen Methoden zur Herstellung von Amidinen aus Benzonitrilen anwenden, wie sie in Houben-Weyl, Methoden der organischen Chemie, E5, S. 1304 f., J. Amer. Chem. Soc. 1957, 427 und J. Org. Chem. 1987, 1017, beschrieben sind.

Die Synthese der Verbindungen III erfolgt nach Schema 4 durch Reaktion eines substituierten Nitrobenzoesäureesters IX mit einem geeigneten Diamin in einem polaren Lösungsmittel wie Dimethylformamid in Gegenwart einer Base wie Kaliumcarbonat bei 100°C bis 150°C, bevorzugt bei 110°C bis 130°C, insbesondere bei etwa 120°C und anschließender Hydrierung in Gegenwart eines geeigneten Katalysators wie 10 % Palladium auf Kohle.

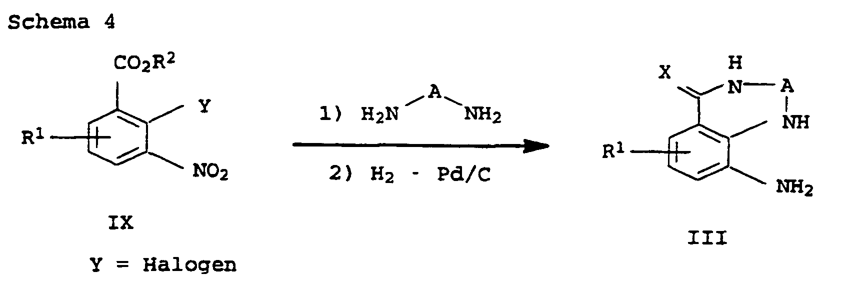

Gegenstand der Erfindung sind außerdem die Zwischenprodukte der Formel III 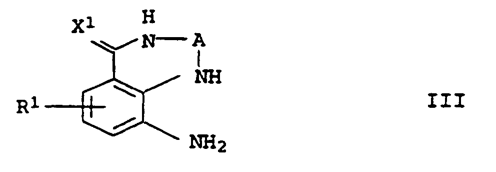 worin
- A: eine Kette C₁-C₃, wobei jedes Kohlenstoff-Atom noch einen oder zwei der folgenden Substituenten tragen kann: C₁-C₄-Alkyl, OH, O-C₁-C₄-Alkyl, CO₂H, CO₂-C₁-C₄-Alkyl und Phenyl oder ein C-Atom auch eine =O-Gruppe tragen kann und
- X¹ und R¹: die in den vorherigen Ansprüchen genannten Bedeutungen haben,
wobei die Verbindungen
9-Amino-3-methyl-1,2,3,4-tetrahydro-5H-1,4-benzodiazepin-5-on,
9-Amino-3-methyl-3,4-dihydro-1H-1,4-benzodiazepin-2,5-dion,
6,8-Diamino-2,4-(1H,3H)-chinazolindion,
8-Amino-2,4-(1H,3H)-chinazolindion
ausgenommen sind,
sowie ihre Salze.

Außerdem ein Verfahren zur Herstellung von Verbindungen der Formel III sowie deren Salze, wobei 2-Halogeno-3-nitro-benzoesäureester mit einem geeigneten Diamin in einem polaren Lösungsmittel in Gegenwart einer Base umgesetzt werden und anschließend die Nitrogruppe mit Wasserstoff in Gegenwart eines geeigneten Katalysators hydriert wird,
und die Verwendung von Verbindungen der Formel III in der Synthese von PARP-Inhibitoren.

Die in der vorliegenden Erfindung enthaltenen substituierten Benzodiazepin-Derivate I stellen Inhibitoren des Enzyms Poly(ADP-ribose)polymerase oder PARP (EC 2.4.2.30) dar.

Die inhibitorische Wirkung der substituierten Benzodiazepin-Derivate I kann mit einem in der Literatur bereits bekannten Enzymtest ermittelt werden, wobei als wirkmaßstab ein Kᵢ-Wert ermittelt wird. Die Benzodiazepin-Derivate I wurden in dieser Weise auf Hemmwirkung des Enzyms Poly(ADP-ribose)polymerase oder PARP (EC 2.4.2.30) gemessen.

Die substituierten Benzodiazepin-Derivate der allgemeinen Formel I stellen Inhibitoren der Poly(ADP-ribose)polymerase (PARP) bzw. wie es auch genannt wird Poly(ADP-ribose)synthase (PARS) dar und können somit zur Behandlung und Prophylaxe von Krankheiten, die mit einer erhöhten Enzymaktivität dieser Enzyme verbunden sind, dienen.

Die Verbindungen der Formeln I können zur Herstellung von Arzneimitteln zur Behandlung von Schädigungen nach Ischämien und zur Prophylaxe bei erwarteten Ischämien verschiedener Organe eingesetzt werden.

Die vorliegenden Benzodiazepin-Derivate der allgemeinen Formel I können danach zur Behandlung und Prophylaxe von neurodegenerativen Krankheiten, die nach Ischämie, Trauma (Schädel-Hirntrauma), Massenblutungen, Subarachnoidal-Blutungen und Stroke auftreten, und von neurodegenerativen Krankheiten wie multipler Infarkt-Dementia, Alzheimer Krankheit, Huntington Krankheit und von Epilepsien, insbesondere von generalisierten epileptischen Anfällen, wie zum Beispiel Petit mal und tonisch-clonische Anfälle und partiell epileptischen Anfällen, wie Temporal Lobe, und komplex-partiellen Anfällen, und weiterhin zur Behandlung und Prophylaxe von Schädigungen des Herzens nach cardialen Ischämien und Schädigungen der Nieren nach renalen Ischämien, zum Beispiel der akuten Niereninsuffizienz, verursacht durch medikamentöse Therapien wie z.B. bei der Cyclosporin-Behandlung, des akuten Nierenversagens oder von Schädigungen, die während und nach einer Nierentransplantation auftreten, dienen. Weiterhin können die Verbindungen der allgemeinen Formel I zur Behandlung des akuten Myocardinfarkts und Schädigungen, die während und nach dessen medikamentöser Lyse auftreten (zum Beispiel mit TPA, Reteplase, Streptokinase oder mechanisch mit einem Laser oder Rotablator) und von Mikroinfarkten während und nach Herzklappenersatz, Aneurysmenresektionen und Herztransplantationen dienen. Ebenfalls können die vorliegenden Benzodiazepin-Derivate I zur Behandlung @@@@einer Revascularisation kritisch verengter Koronaraterien, zum Beispiel bei der PCTA und Bypass-Operationen, und kritisch verengter peripherer Arterien, zum Beispiel Beinarterien, dienen. Zudem können die Benzodiazepin-Derivate I zur Behandlung von Tumoren und deren Metastasierung nützlich sein und zur Behandlung von Entzündungen und rheumatischen Erkrankungen, wie z.B. rheumatischer Arthritis und auch zur Behandlung von Diabetes mellitus dienen, zur Behandlung des Multiorganversagens z.B. beim septischen Schock und zur Behandlung des ARDS ("acute respiratory distress-syndrom" Schocklunge).

Die erfindungsgemäßen Arzneimittelzubereitungen enthalten neben den üblichen Arzneimittelhilfsstoffen eine therapeutisch wirksame Menge der Verbindungen I.

Für die lokale äußere Anwendung, zum Beispiel in Puder, Salben oder Sprays, können die Wirkstoffe in den üblichen Konzentrationen enthalten sein. In der Regel sind die wirkstoffe in einer Menge von 0,001 bis 1 Gew.-%, vorzugsweise 0,001 bis 0,1 Gew.-% enthalten.

Bei der inneren Anwendung werden die Präparationen in Einzeldosen verabreicht. In einer Einzeldosis werden pro kg Körpergewicht 0,1 bis 100 mg gegeben. Die Zubereitung können täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes Hydriertes Ricinusöl, Polyacrylsäure, Polyethylenglykol, Polyethylenglykolstearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wirkstoff verträglich. Die Herstellung der Arzneimittelzubereitungen erfolgt in üblicher Weise, zum Beispiel durch Vermischung des Wirkstoffes mit üblichen Trägerstoffen und Verdünnungsmitteln.

Die Arzneimittelzubereitungen können in verschiedenen Applikationsweisen verabreicht werden, zum Beispiel peroral, parenteral wie intravenös durch Infusion, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

### Pharmakologisches Beispiel:

Hemmung des Enzyms Poly(ADP-ribose)polymerase oder PARP (EC 2.4.2.30)

Eine 96well Mikrotiterplatte (Flacon) wird mit Histonen (Type II-AS; SIGMA H7755) beschichtet. Histone werden dazu in Carbonat-Puffer (0,05 M NaHCO₃; pH 9,4) zu einer Konzentration von 50 µg/ml gelöst. Die einzelnen Wells der Mikrotiterplatten werden über Nacht mit je 100 µl dieser Histon Lösung inkubiert. Anschließend wird die Histon Lösung entfernt und die einzelnen Wells mit 200 µl einer 1%igen BSA (Bovine Serum Albumine) Lösung in Carbonat-Puffer für 2 Stunden bei Raumtemperatur inkubiert. Anschließend wird dreimal mit Waschpuffer (0,05 % Tween10 in PBS) gewaschen. Für die Enzymreaktion werden je Well 50 µl der Enzymreaktionslösung (5 µl Reaktions-Puffer (1 M Tris-HCl pH 8,0, 100 mM MgCl₂, 10 mM DTT), 0,5 µl PARP (c = 0,22 µg/µl), 4 µl aktivierte DNA (SIGMA D-4522, 1 mg/ml in Wasser), 40,5 µl H₂O) mit 10 µl einer Inhibitorlösung für 10 Minuten vorinkubiert. Die Enzymreaktion wird durch Zugabe von 40 µl einer Substratlösung (4 µl Reaktion-Puffer (s.o.), 8 µl NAD-Lösung (100 µM in H₂O), 28 µl H₂O) gestartet. Reaktionszeit ist 20 Minuten bei Raumtemperatur. Die Reaktion wird durch dreimaliges Waschen mit Waschpuffer (s.o.) gestoppt. Anschließend folgt eine einstündige Inkubation bei Raumtemperatur mit einem spezifischen Anti-Poly-ADP-Ribose Antikörper inkubiert. Als Antikörper wurde ein monoklonaler anti-Poly-(ADP-ribose) Antikörpern "10H" (Kawarnaitsu H et al. (1984) Monoclonal antibodies to poly (adenosine diphosphate ribose) recognize different structures. Biochemistry 23, 3771-3777) verwendet. Polyklonale Antikörper können ebenso verwendet werden.

Die Antikörper wurden in einer 1:5000 Verdünnung in AntikörperPuffer (1 % BSA in PBS; 0,05 % Tween20) eingesetzt. Nach dreimaligem Waschen mit Waschpuffer folgt eine einstündige Inkubation bei Raumtemperatur mit dem sekundären Antikörper. Hier wurden für den monoklonalen Antikörper ein anti-Maus-IgG gekoppelt mit Peroxidase (Boehringer Mannheim) und für den Kaninchen Antikörper ein anti-Rabbit-IgG gekoppelt mit Peroxidase (SIGMA A-6154) jeweils in einer 1:10.000 Verdünnung in Antikörperpuffer verwendet. Nach dreimaligem Waschen mit Waschpuffer erfolgt die Farbreaktion unter Verwendung von 100 µl/Well Farbreagenz (SIGMA, TMB-Fertigmix, T8540) für ca. 15 min. bei Raumtemperatur. Die Farbreaktion wird durch Zugabe von 100 µl 2 M H₂SO₄ gestoppt. Danach wird sofort gemessen (450 nm gegen 620nm; ELISA Platten Lesegerät "Easy Reader" EAR340AT, SLT-Labinstruments, Österreich). Der IC50-Wert eines zu messenden Inhibitors liegt bei der Inhibitorkonzentration, wo eine halbmaximale Farbkonzentrationsänderung auftritt.

### Beispiele

### Beispiel 11

2-(Piperidin-4-yl)-5,6-dihydroimidazo[4,5,1-*jk*][1,4]benzodiazepin-7(4*H*)-on × HCl
[M⁺ = 271]

### Beispiel 12

2-(1-n-Propyl-piperidin-4-yl)-5,6-dihydroimidazo[4,5,1-*jk*]-[1,4]benzodiazepin-7(4*H*)-on × HCl
[M⁺ = 313]

### Beispiel 13

2-(1-Benzyl-piperidin-4-yl)-5,6-dihydroimidazo[4,5,1-*jk*][1,4]-benzodiazepin-7(4*H*)-on × HCl
[M⁺ = 361]

### Beispiel 30

2-(1-(1-Methyl-piperidin-4-yl)-piperidin-4-yl)-5,6-dihydroimidazo[4,5,1-*jk*][1,4]benzodiazepin-7(4*H*)-on × 2 HCl
[M⁺ = 369]

### Beispiel 32

2-(1-Benzyl-piperidin-3-yl)-5,6-dihydroimidazo[4,5,1-*jk*][1,4]-benzodiazepin-7(4*H*)-on
[M⁺ = 360]

### Beispiel 81

2-(6-Nitro-1,3-benzodioxol-5-yl)-5,6-dihydroimidazo[4,5,1-*jk*]-[1,4]benzodiazepin-7(4*H*)-on
[M⁺-1 = 352]

### Beispiel 82

2-(2,3-Dihydro-1,4-benzodioxin-6-yl)-5,6-dihydroimidazo-[4,5,1-*jk*][1,4]benzodiazepin-7(4*H*)-on
[M⁺-1 = 321]

### Beispiel 110

2-(1,3-Benzodioxol-5-yl)-5,6-dihydroimidazo[4,5,1-*jk*][1,4]benzodiazepin-7(4*H*)-on
[M⁺-1 = 307]

### Beispiel 154

2-(2,5-Dimethoxytetrahydro-3-furanyl)-5,6-dihydroimidazo-[4,5,1-*jk*][1,4]benzodiazepin-7(4*H*)-on
[M⁺-1 = 317]

### Beispiel 170

2-(2,3-Dihydro-1-benzofuran-5-yl)-5,6-dihydroimidazo[4,5,1-*jk*]-[1,4]benzodiazepin-7(4*H*)-on
[M⁺-1 = 305]

### Beispiel 215

2-(6-Chloro-1,3-benzodioxol-5-yl)-5,6-dihydroimidazo[4,5,1-*jk*]-[1,4]benzodiazepin-7(4*H*)-on
[M⁺-1 = 341]

Folgende erfindungsgemäße Verbindungen können analog den oben beschriebenen Methoden hergestellt werden:
28. 2-Piperidin-4-yl-5,6-dihydro-imidazo[4,5,1-jk][1,4]-benzodiazepin-7(4H)-on
29. 2-(1-Ethyl-piperidin-4-yl)-5,6-dihydro-imidazo[4,5,1-jk]-[1,4]benzodiazepin-7(4H)-on
30. 2-(1-n-Propyl-piperidin-4-yl)-5,6-dihydro-imidazo[4,5,1-jk]-[1,4]benzodiazepin-7(4H)-on
31. 2-(1-Isopropyl-piperidin-4-yl)-5,6-dihydro-imidazo[4,5,1-jk]-[1,4]benzodiazepin-7(4H)-on

## Patentansprüche

1. Verbindungen der Formel I 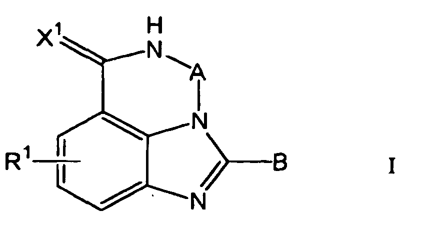 worin
A eine C₁-C₃-Kohlenwasserstoffkette ist, wobei jedes Kohlenstoffatom noch einen oder zwei der folgenden Substituenten tragen kann:
C₁-C₄-Alkyl, OH, O-C₁-C₄-Alkyl, COOH, COO-C₁-C₄-Alkyl und Phenyl oder ein C-Atom auch eine =O -Gruppe tragen kann und
X¹ S, O oder NH ist und
R¹ Wasserstoff, Chlor, Fluor, Brom, Iod, verzweigtes oder unverzweigtes C₁-C₆-Alkyl, OH, Nitro, CF₃, CN, NR¹¹R¹², NH-CO-R¹³ oder O-C₁-C₄-Alkyl ist, wobei R¹¹ und R¹² unabhängig voneinander Wasserstoff oder C₁-C₄-Alkyl bedeuten und R¹³ Wasserstoff, C₁-C₄-Alkyl, C₁-C₄-Alkyl-Phenyl oder Phenyl bedeutet, und
B Piperidin oder Piperazin, die noch mit einen R⁴ oder maximal 2 R⁵ substituiert sein können, bedeutet, und
R⁴ Wasserstoff oder -(D)ₚ-(E)ₛ- (F¹)_{q}-G¹-(F²)ᵣ-(G²)-G³ bedeutet, wobei
D S, NR⁴³ oder O
E Phenyl, 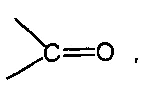 -SO₂-, -SO₂NH-, -NHCO-, -CONH-, -NHSO₂-, -NHCOCH₂X⁴-, und
X⁴ S, O oder NH bedeutet, und
F¹ eine geradkettige oder verzweigte gesättigte oder ungesättigte Kohlenstoffkette mit 1 bis 8 C-Atomen ist und
F² unabhängig von F¹ die gleiche Bedeutung wie F¹ besitzt,
G¹ eine Bindung bedeutet oder einen ungesättigten, gesättigten oder partialungesättigten mono-, bi- oder tricyclischen Ring mit maximal 15 Kohlenstoffatomen, einen ungesättigten, gesättigten oder partial-ungesättigten mono-, bi- oder tricyclischen Ring mit maximal 14 Kohlenstoffatomen und 0 bis 5 Stickstoffatomen, 0 bis 2 Sauerstoffatomen bzw. 0 bis 2 Schwefelatomen bedeutet, die jeweils noch mit maximal 3 unterschiedlichen oder gleichen Resten R⁵ substituiert sind, und ein oder zwei Kohlenstoff- bzw. Schwefelatome auch ein oder zwei =O -Gruppen tragen können, und
G² bedeutet a) NR⁴¹R⁴² oder 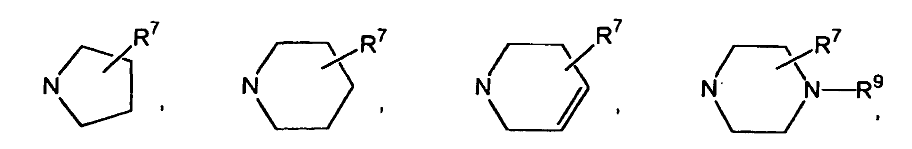 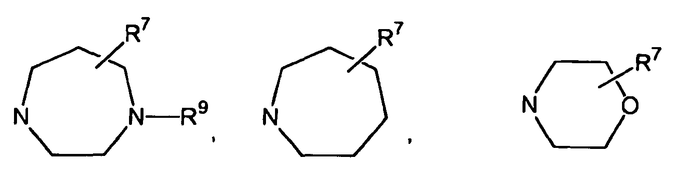 dann ist G³ Wasserstoff,
oder G² ist b) eine Bindung, dann bedeutet
G³ einen ungesättigten, gesättigten oder partial-ungesättigten mono-, bi- oder tricyclischen Ring mit maximal 15 Kohlenstoffatomen, einen ungesättigten, gesättigten oder partial-ungesättigten mono-, bi-oder tricyclischen Ring mit maximal 14 Kohlenstoffatomen und 0 bis 5 Stickstoffatomen, 0 bis 2 Sauerstoffatomen bzw. 0 bis 2 Schwefelatomen bedeutet, die jeweils noch mit maximal 3 unterschiedlichen oder gleichen Resten R⁵ substituiert sind, und ein oder zwei Kohlenstoff- bzw. Schwefelatome auch ein oder zwei =O -Gruppen tragen können, oder Wasserstoff bedeutet, und
p 0 oder 1 bedeutet und
s 0 oder 1 und
q 0 oder 1 sein kann und
r 0 oder 1 sein kann und
R⁴¹ Wasserstoff, C₁-C₆-Alkyl, wobei jedes Kohlenstoffatom noch bis zu zwei Reste R⁶ tragen kann, Phenyl, der noch maximal zwei Reste R⁶ tragen kann, oder (CH₂)ₜ-K ist, und
R⁴² Wasserstoff, C₁-C₆-Alkyl, CO-R⁸, CO₂-R⁸, SO₂NH₂, SO₂-R⁸, -(C=NH)-R⁸ oder -(C=NH)-NHR⁸ ist, und
R⁴³ Wasserstoff oder C₁-C₄-Alkyl, und
t 1, 2, 3 oder 4 ist und
K NR¹¹R¹², NR¹¹-C₁-C₄-Alkyl-Phenyl, Pyrrolidin, Piperidin, 1.2.5.6-Tetrahydropyridin, Morpholin, Homopiperidin, Piperazin, das noch mit einem Alkyl-Rest C₁-C₆-Alkyl substituiert sein kann, oder Homopiperazin, das noch mit einem Alkyl-Rest C₁-C₆-Alkyl substituiert sein kann, und
R⁵ Wasserstoff, Chlor, Fluor, Brom, Iod, OH, Nitro, CF₃, CN, NR¹¹R¹², NH-CO-R¹³, C₁-C₄-Alkyl-CO-NH-R¹³, COR⁸, C₀-C₄-Alkyl-O-CO-R¹³, C₁-C₄-Alkyl-Phenyl, Phenyl, CO₂-C₁-C₄-Alkyl, verzweigtes oder unverzweigtes C₁-C₆-Alkyl, O-C₁-C₄-Alkyl, S-C₁-C₄-Alkyl ist, wobei jedes C-Atom der Alkylketten bis zu zwei Reste R⁶ tragen kann und die Alkylketten auch ungesättigt sein können,und
R⁶ Wasserstoff, Chlor, Fluor, Brom, Iod, verzweigtes oder unverzweigtes C₁-C₆-Alkyl, OH, Nitro, CF₃, CN, NR¹¹R¹², NH-CO-R¹³, O-C₁-C₄-Alkyl ist, und
R⁷ Wasserstoff, C₁-C₆-Alkyl, Phenyl, wobei der Ring noch mit bis zu zwei Resten R⁷¹ substituiert sein kann, ein Amin NR¹¹R¹² oder ein zyklisches gesättigtes Amin mit 3 bis 7 Gliedern, das noch mit einem Alkyl-Rest C₁-C₆-Alkyl substituiert sein kann, oder Homopiperazin, das noch mit einem Alkyl-Rest C₁-C₆-Alkyl substituiert sein kann, und
wobei die Reste R¹¹, R¹² und R¹³ in K, R⁵, R⁶ und R⁷ unabhängig voneinander die gleiche Bedeutung annehmen können wie R¹, und
R⁷¹ OH, C₁-C₆-Alkyl, O-C₁-C₄-Alkyl, Chlor, Brom, Iod, Fluor, CF₃, Nitro oder NH₂ ist, und
R⁸ C₁-C₆-Alkyl, CF₃, Phenyl oder C₁-C₄-Alkyl-Phenyl ist, wobei der Ring noch mit bis zu zwei Resten R⁸¹ substituiert sein kann, und
R⁸¹ OH, C₁-C₆-Alkyl, O-C₁-C₄-Alkyl, Chlor, Brom, Iod, Fluor, CF₃, Nitro oder NH₂ ist, und
R⁹ Wasserstoff, C₁-C₆-Alkyl, C₁-C₄-Alkyl-Phenyl, CO₂-C₁-C₄-Alkyl-Phenyl, CO₂-C₁-C₄-Alkyl, SO₂-Phenyl, COR⁸ oder Phenyl ist, wobei die Phenyl-Ringe noch mit bis zu zwei Resten R⁹¹ substituiert sein können, und
R⁹¹ OH, C₁-C₆-Alkyl, O-C₁-C₄-Alkyl, Chlor, Brom, Iod, Fluor, CF₃, Nitro oder NH₂ ist,
sowie ihre tautomeren Formen, möglichen enantiomeren und diastereomeren Formen, und deren Prodrugs.

2. Verbindungen der Formel I nach Anspruch 1, wobei
A eine C₂-Kohlenwasserstoffkette ist, die substituiert sein kann, und
X¹ O darstellt und
R¹ Wasserstoff ist.

3. Verbindungen der Formel I nach einem der Ansprüche 1 oder 2, wobei
R⁴ Wasserstoff oder D_{0.1}-F¹_{0.1}-G²-G³ mit G³ gleich Wasserstoff bedeutet und
D O oder NR⁴³, wobei R⁴³ Wasserstoff oder C₁-C₃-Alkyl ist, und
F¹ C₂-C₄-Alkyl bedeutet.

4. Verbindungen ausgewählt aus der Gruppe bestehend aus 2-(6-Nitro-1,3-benzodioxol-5yl)-5,6-dihydroimidazo[4,5,1*-jk*][1,4]benzodiazepin-7(4H)-on, 2-(2,3-Dihydro-1,3-benzodioxin-6-yl)-5,6-dihydroimidazo[4,5,1-*jk*][1,4]benzodiazepin-7(4H)-on, 2-(1,3-Benzodioxol-5-yl)-5,6-dihydroimidazo[4,5,1-*jk*][1,4]benzodiazepin-7(4H)-on, 2-(2,5-Dimethoxytetrahydro-3-furanyl)-5,6-dihydroimidazo[4,5,1-*jk*][1,4] benzodiazepin-7(4H)-on, 2-(2,3-Diyhdro-1-benzofuran-5-yl)-5,6-dihydroimidazo [4,5,1-*jk*][1,4]benzodiazepin-7(4H)-on, 2-(6-Chlor-1,3-benzodioxol-5-yl)-5,6-dihydroimidazo[4,5,1-*jk*][1,4]benzodiazepin-7(4H)-on sowie ihre tautomeren Formen, möglichen enantiomeren und diastereomeren Formen, und deren Prodrugs.

5. Arzneimittel enthaltend neben üblichen Träger und Hilfsstoffen Verbindungen der Formel I nach einem der Ansprüche 1 bis 4.

6. Verwendung von Verbindungen der allgemeinen Formel I nach einem der Ansprüchen 1 bis 4 zur Herstellung von Arzneimitteln mit PARP-inhibierender Wirkung.

7. Verwendung von Verbindungen der Formel I nach Anspruch 6 zur Herstellung von Arzneimitteln zur Behandlung von neuro-degenerativen Krankheiten und neuronalen Schädigungen.

8. Verwendung nach Anspruch 6 zur Behandlung von solchen neuro-degenerativen Krankheiten und neuronalen Schädigungen, die durch Ischämie, Trauma oder Massenblutungen ausgelöst werden.

9. Verwendung nach Anspruch 6 zur Behandlung des Schlaganfalls und des Schädel-Hirntraumas.

10. Verwendung nach Anspruch 6 zur Behandlung der Alzheimerschen Krankheit, der Parkinsonsche Krankheit und der Huntington-Krankheit.

11. Verwendung von Verbindungen der Formel I nach Anspruch 6 zur Herstellung von Arzneimitteln zur Behandlung oder Prophylaxe von Schädigungen durch Ischämien.

12. Verwendung von Verbindungen der Formel I nach Anspruch 6 zur Herstellung von Arzneimitteln zur Behandlung von Epilepsien, insbesondere von generalisierten epileptischen Anfällen, wie zum Beispiel Petit mal und tonisch-clonische Anfälle und partiell epileptischen Anfällen, wie Temporal Lobe, und komplex-partiellen Anfällen.

13. Verwendung von Verbindungen der Formel I nach Anspruch 6 zur Herstellung von Arzneimitteln zur Behandlung von Schädigungen der Nieren nach renalen Ischämien, Schädigungen, die durch medikamentöse Therapie verursacht werden, wie zum Beispiel während der Cyclosporin-Therapie, und zur Behandlung während und nach Nierentransplantationen.

14. Verwendung von Verbindungen der Formel I nach Anspruch 6 zur Herstellung von Arzneimitteln zur Behandlung von Schädigungen des Herzens nach cardialen Ischämien.

15. Verwendung von Verbindungen der Formel I nach Anspruch 6 zur Herstellung von Arzneimitteln zur Behandlung von Mikroinfarkten wie zum Beispiel während und nach Herzklappenersatz, Aneurysmenresektionenen und Herztransplantationen.

16. Verwendung von Verbindungen der Formel I nach Anspruch 6 zur Herstellung von Arzneimitteln zur Behandlung bei einer Revascularisation kritisch verengter Koronararterien wie zum Beispiel bei PTCA und Bypass-Operationen oder kritisch verengter peripherer Arterien, insbesondere Beinarterien.

17. Verwendung von Verbindungen der Formel I nach Anspruch 6 zur Herstellung von Arzneimitteln zur Behandlung des akuten Myocardinfarktes und von Schädigungen während und nach dessen medikamentöser oder mechanischer Lyse.

18. Verwendung von Verbindungen der Formel I nach Anspruch 6 zur Herstellung von Arzneimitteln zur Behandlung von Tumoren und deren Metastasierung.

19. Verwendung von Verbindungen der Formel I nach Anspruch 6 zur Herstellung von Arzneimitteln zur Behandlung von Sepsis, des Multiorganversagens wie zum Beispiel wahrend des septischen Schocks und des "acute respiratory distresssyndroms".

20. Verwendung von Verbindungen der Formel I nach Anspruch 6 zur Herstellung von Arzneimitteln zur Behandlung von immunologischen Krankheiten wie Entzündungen und rheumatische Erkrankungen, wie zum Beispiel rheumatoide Arthritis.

21. Verwendung von Verbindungen der Formel I nach Anspruch 6 zur Herstellung von Arzneimitteln zur Behandlung von Diabetes mellitus.

## Claims

1. Compounds of formula I 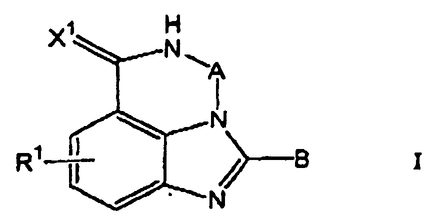 wherein
A is a C₁-C₃-hydrocarbon chain, wherein each carbon atom may further carry one or two of the following substituents:
C₁-C₄-alkyl, OH, O-C₁-C₄-alkyl, COOH, COO-C₁-C₄-alkyl and phenyl, or one carbon atom may also carry a =O group, and
X¹ is S, O or NH and
R¹ is hydrogen, chlorine, fluorine, bromine, iodine, branched or unbranched C₁-C₆-alkyl, OH, nitro, CF₃, CN, NR¹¹R¹², NH-CO-R¹³ or O-C₁-C₄-alkyl, wherein R¹¹ and R¹² independently of one another represent hydrogen or C₁-C₄-alkyl and R¹³ represents hydrogen, C₁-C₄-alkyl, C₁-C₄-alkylphenyl or phenyl, and
B represents piperidine or piperazine, which may further be substituted by one R⁴ or by not more than 2 R⁵, and
R⁴ represents hydrogen or -(D)ₚ-(E)ₛ-(F¹)_{q}-G¹-(F²)ᵣ-(G²)-G³, wherein
D represents S, NR⁴³ or O
E represents phenyl, >C=O, -SO₂-, -SO₂NH-, -NHCO-, -CONH-, -NHSO₂-, -NHCOCH₂X⁴-, and
X⁴ represents S, O or NH, and
F¹ is a straight-chain or branched saturated or unsaturated carbon chain having from 1 to 8 carbon atoms and
F² independently of F¹ has the same meaning as F¹,
G¹ represents a bond or represents an unsaturated, saturated or partially unsaturated mono-, bi- or tri-cyclic ring having not more than 15 carbon atoms, or an unsaturated, saturated or partially unsaturated mono-, bi- or tri-cyclic ring having not more than 14 carbon atoms and from 0 to 5 nitrogen atoms, from 0 to 2 oxygen atoms or from 0 to 2 sulfur atoms, each of which is further substituted by not more than 3 different or identical radicals R⁵, and one or two carbon or sulfur atoms may also carry one or two =O groups, and
G² represents a) NR⁴¹R⁴² or 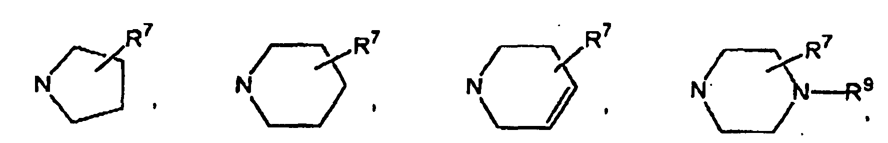 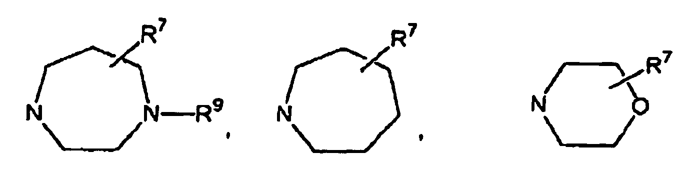 in which case G³ is hydrogen,
or G² is b) a bond, in which case
G³ represents an unsaturated, saturated or partially unsaturated mono-, bi- or tri-cyclic ring having not more than 15 carbon atoms, or an unsaturated, saturated or partially unsaturated mono-, bi- or tri-cyclic ring having not more than 14 carbon atoms and from 0 to 5 nitrogen atoms, from 0 to 2 oxygen atoms or from 0 to 2 sulfur atoms, each of which is further substituted by not more than 3 different or identical radicals R⁵, and one or two carbon or sulfur atoms may also carry one or two =O groups, or G³ represents hydrogen, and
p represents 0 or 1, and
s may be 0 or 1 and
q may be 0 or 1 and
r may be 0 or 1 and
R⁴¹ is hydrogen, C₁-C₆-alkyl, wherein each carbon atom may further carry up to two radicals R⁶, phenyl, which may further carry not more than two radicals R⁶, or (CH₂)ₜ-K, and
R⁴² is hydrogen, C₁-C₆-alkyl, CO-R⁸, CO₂-R⁸, SO₂NH₂, SO₂-R⁸, -(C=NH)-R⁸ or -(C=NH)-NHR⁸, and
R⁴³ is hydrogen or C₁-C₄-alkyl and
t is 1, 2, 3 or 4 and
K is NR¹¹R¹², NR¹¹-C₁-C₄-alkyl-phenyl, pyrrolidine, piperidine, 1,2,5,6-tetrahydropyridine, morpholine, homopiperidine, piperazine, which may further be substituted by an alkyl radical C₁-C₆-alkyl, or homopiperazine, which may further be substituted by an alkyl radical C₁-C₆-alkyl, and
R⁵ is hydrogen, chlorine, fluorine, bromine, iodine, OH, nitro, CF₃, CN, NR¹¹R¹², NH-CO-R¹³, C₁-C₄-alkyl-CO-NH-R¹³, COR⁸, C₀-C₄-alkyl-O-CO-R¹³, C₁-C₄-alkyl-phenyl, phenyl, CO₂-C₁-C₄-alkyl, branched or unbranched C₁-C₆-alkyl, O-C₁-C₄-alkyl, S-C₁-C₄-alkyl, wherein each carbon atom of the alkyl chains may carry up to two radicals R⁶ and the alkyl chains may also be unsaturated, and
R⁶ is hydrogen, chlorine, fluorine, bromine, iodine, branched or unbranched C₁-C₆-alkyl, OH, nitro, CF₃, CN, NR¹¹R¹², NH-CO-R¹³, O-C₁-C₄-alkyl, and
R⁷ is hydrogen, C₁-C₆-alkyl, phenyl, wherein the ring may further be substituted by up to two radicals R⁷¹, an amine NR¹¹R¹² or a cyclic saturated amine having from 3 to 7 members, which may further be substituted by an alkyl radical C₁-C₆-alkyl, or homopiperazine, which may further be substituted by an alkyl radical C₁-C₆-alkyl, and
wherein the radicals R¹¹, R¹² and R¹³ in K, R⁵, R⁶ and R⁷ may, independently of one another, have the same meanings as R¹, and
R⁷¹ is OH, C₁-C₆-alkyl, O-C₁-C₄-alkyl, chlorine, bromine, iodine, fluorine, CF₃, nitro or NH₂, and
R⁸ is C₁-C₆-alkyl, CF₃, phenyl or C₁-C₄-alkylphenyl, wherein the ring may further be substituted by up to two radicals R⁸¹, and
R⁸¹ is OH, C₁-C₆-alkyl, O-C₁-C₄-alkyl, chlorine, bromine, iodine, fluorine, CF₃, nitro or NH₂, and
R⁹ is hydrogen, C₁-C₆-alkyl, C₁-C₄-alkyl-phenyl, CO₂-C₁-C₄-alkyl-phenyl, CO₂-C₁-C₄-alkyl, SO₂-phenyl, COR⁸ or phenyl, wherein the phenyl rings may further be substituted by up to two radicals R⁹¹, and
R⁹¹ is OH, C₁-C₆-alkyl, O-C₁-C₄-alkyl, chlorine, bromine, iodine, fluorine, CF₃, nitro or NH₂,
and their tautomeric forms, possible enantiomeric and diastereoisomeric forms, and prodrugs thereof.

2. Compounds of formula I according to claim 1, wherein
A is a C₂-hydrocarbon chain, which may be substituted, and
X¹ represents O and
R¹ is hydrogen.

3. Compounds of formula I according to either claim 1 or claim 2, wherein
R⁴ represents hydrogen or D_{0,1}-F¹_{0,1}-G²-G³, wherein G³ is hydrogen, and
D represents O or NR⁴³, wherein R⁴³ is hydrogen or C₁-C₃-alkyl, and
F¹ represents C₂-C₄-alkyl.

4. Compounds selected from the group consisting of 2-(6-nitro-1,3-benzodioxol-5-yl)-5,6-dihydroimidazo-[4,5,1-*jk*][1,4]benzodiazepin-7(4H)-one, 2-(2,3-dihydro-1,3-benzodioxin-6-yl)-5,6-dihydroimidazo-[4,5,1-*jk*][1,4]benzodiazepin-7(4H)-one, 2-(1,3-benzodioxol-5-yl)-5,6-dihydroimidazo-[4,5,1-*jk*][1,4]benzodiazepin-7(4H)-one, 2-(2,5-dimethoxytetrahydro-3-furanyl)-5,6-dihydroimidazo-[4,5,1-*jk*][1,4]benzodiazepin-7(4H)-one, 2-(2,3-dihydro-1-benzofuran-5-yl)-5,6-dihydroimidazo-[4,5,1-*jk*][1,4]benzodiazepin-7(4H)-one, 2-(6-chloro-1,3-benzodioxol-5-yl)-5,6-dihydroimidazo-[4,5,1-*jk*][1,4]benzodiazepin-7(4H)-one,
and their tautomeric forms, possible enantiomeric and diastereoisomeric forms, and prodrugs thereof.

5. Medicament comprising, in addition to conventional carriers and auxiliary substances, compounds of formula I according to any one of claims 1 to 4.

6. Use of compounds of the general formula I according to any one of claims 1 to 4 in the preparation of medicaments having PARP-inhibiting action.

7. Use of compounds of formula I according to claim 6 in the preparation of medicaments for the treatment of neurodegenerative diseases and neuronal damage.

8. Use according to claim 6 in the treatment of neurodegenerative diseases and neuronal damage caused by ischaemia, trauma or massive bleeding.

9. Use according to claim 6 in the treatment of stroke and craniocerebral trauma.

10. Use according to claim 6 in the treatment of Alzheimer's disease, Parkinson's disease and Huntington's disease.

11. Use of compounds of formula I according to claim 6 in the preparation of medicaments for the treatment or prophylaxis of damage caused by ischaemias.

12. Use of compounds of formula I according to claim 6 in the preparation of medicaments for the treatment of epilepsy, in particular of generalised epileptic seizures, such as, for example, petit mal and tonoclonic seizures, and partial epileptic seizures, such as temporal lobe, and complex partial seizures.

13. Use of compounds of formula I according to claim 6 in the preparation of medicaments for the treatment of damage to the kidneys following renal ischaemias, damage caused by drug therapy, such as, for example, during cyclosporin therapy, and for treatment during and after kidney transplants.

14. Use of compounds of formula I according to claim 6 in the preparation of medicaments for the treatment of damage to the heart following cardiac ischaemias.

15. Use of compounds of formula I according to claim 6 in the preparation of medicaments for the treatment of microinfarcts, such as, for example, during and after heart valve replacement, aneurysm resections and heart transplants.

16. Use of compounds of formula I according to claim 6 in the preparation of medicaments for treatment in the revascularisation of critically narrowed coronary arteries, such as, for example, in PTCA and bypass operations, or of critically narrowed peripheral arteries, especially leg arteries.

17. Use of compounds of formula I according to claim 6 in the preparation of medicaments for the treatment of acute myocardial infarct and of damage during and after medical or mechanical lysis thereof.

18. Use of compounds of formula I according to claim 6 in the preparation of medicaments for the treatment of tumours and the metastasis thereof.

19. Use of compounds of formula I according to claim 6 in the preparation of medicaments for the treatment of sepsis, of multiorgan failure, such as, for example, during septic shock and acute respiratory distress syndrome.

20. Use of compounds of formula I according to claim 6 in the preparation of medicaments for the treatment of immunological diseases such as inflammations and rheumatic disorders, such as, for example, rheumatoid arthritis.

21. Use of compounds of formula I according to claim 6 in the preparation of medicaments for the treatment of diabetes mellitus.

## Revendications

1. Composés de formule I 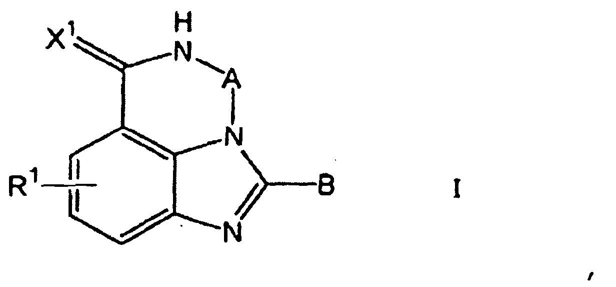 dans laquelle
A est une chaîne d'hydrocarbures en C₁-C₃, chaque atome de carbone pouvant encore porter un à deux des substituants suivants :
alkyle en C₁-C₄, OH, O-alkyle en C₁-C₄, COOH, COO-alkyle en C₁-C₄- et phényle ou un atome de C pouvant également porter un groupe =O et
X¹ est S, O ou NH et
R¹ est hydrogène, chlore, fluor, brome, iode, alkyle en C₁-C₆ ramifié ou non ramifié, OH, nitro, CF₃, CN, NR¹¹R¹², NH-CO-R¹³ ou O-alkyle en C₁-C₄, R¹¹ et R¹² signifiant indépendamment l'un de l'autre hydrogène ou alkyle en C₁-C₄ et R¹³ signifiant hydrogène, alkyle en C₁-C₄, alkylphényle en C₁-C₄ ou phényle, et
B signifie pipéridine ou pipérazine, qui peuvent encore être substituées par un R⁴ ou 2 R⁵ au maximum, et
R⁴ signifie hydrogène ou -(D)ₚ-(E)ₛ-(F¹)_{q}-G¹-(F²)ᵣ-(G²)-G³,
D signifiant S, NR⁴³ ou O
E signifiant phényle, 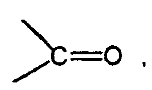 -SO₂-, -SO₂NH-, -NHCO-, -CONH-, -NHSO₂-, -NHCOCH₂X⁴-, et
X₄ signifiant S, O ou NH, et
F¹ étant une chaîne de carbone linéaire ou ramifiée, saturée ou insaturée avec 1 à 8 atomes de C et
F² possédant indépendamment de F¹ la même signification que F¹,
G¹ signifiant une liaison ou un composé mono-, bi- ou tricyclique insaturé, saturé ou partiellement insaturé avec 15 atomes de carbone au maximum, un composé mono-, bi- ou tricyclique insaturé, saturé ou partiellement insaturé avec 14 atomes de carbone au maximum et 0 à 5 atomes d'azote, 0 à 2 atomes d'oxygène ou 0 à 2 atomes de soufre qui sont encore substitués à chaque fois par 3 radicaux R⁵ différents ou identiques au maximum, et un ou deux atomes de carbone ou de soufre pouvant également porter un ou deux groupes =O, et G² signifiant a) NR⁴¹R⁴² ou 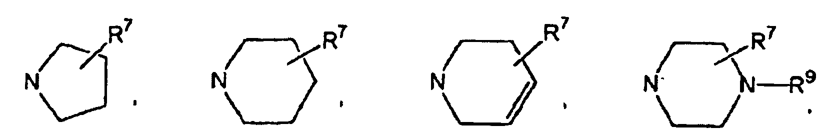 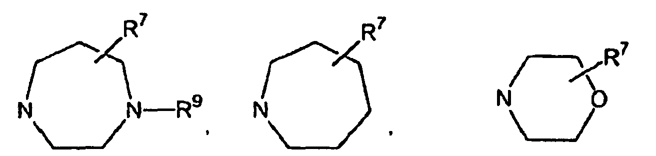 G³ est alors hydrogène,
ou G² est b) une liaison,
G³ signifiant alors un composé mono-, bi- ou tricyclique insaturé, saturé ou partiellement insaturé avec 15 atomes de carbone au maximum, un composé mono-, bi- ou tricyclique insaturé, saturé ou partiellement insaturé avec 14 atomes de carbone au maximum et 0 à 5 atomes d'azote, 0 à 2 atomes d'oxygène ou 0 à 2 atomes de soufre qui sont encore substitués à chaque fois par 3 radicaux R⁵ différents ou identiques au maximum, et un ou deux atomes de carbone ou de soufre pouvant également porter un ou deux groupes =O, ou signifiant hydrogène, et
p signifiant 0 ou 1 et
s étant 0 ou 1 et
q pouvant être 0 ou 1 et
r pouvant être 0 ou 1 et
R⁴¹ étant hydrogène, alkyle en C₁-C₆, chaque atome de carbone pouvant encore porter jusqu'à deux radicaux R⁶, phényle, qui peut encore porter deux radicaux R⁶ au maximum, ou (CH₂)ₜ-K, et
R⁴² étant hydrogène, alkyle en C₁-C₆, CO-R⁸, CO₂-R⁸, SO₂NH₂, SO₂-R⁸, -(C=NH)-R⁸ ou -(C=NH)-NHR⁸, et
R⁴³ étant hydrogène ou alkyle en C₁-C₄, et
t étant 1,2, 3 ou 4 et
K étant NR¹¹R¹², NR¹¹-alkylphényle en C₁-C₄, pyrrolidine, pipéridine, 1,2,5,6-tétrahydropyridine, morpholine, homopipéridine, pipérazine, qui peut encore être substituée par un radical alkyle, alkyle en C₁-C₆, ou homopipérazine, qui peut encore être substituée par un radical alkyle, alkyle en C₁-C₆, et
R₅ étant hydrogène, chlore, fluor, brome, iode, OH, nitro, CF₃, CN, NR¹¹R¹², NH-CO-R¹³, alkyle en C₁-C₄-CO-NH-R¹³, COR⁸, alkyle en C₀-C₄-O-CO-R¹³, alkylphényle en C₁-C₄, phényle, CO₂-alkyle en C₁-C₄, alkyle en C₁-C₆ ramifié ou non ramifié, O-alkyle en C₁-C₄, S-alkyle en C₁-C₄, chaque atome de C des chaînes alkyle pouvant porter jusqu'à deux radicaux R⁶ et les chaînes alkyliques pouvant également être insaturées, et
R⁶ étant hydrogène, chlore, fluor, brome, iode, alkyle en C₁-C₆ ramifié ou non ramifié, OH, nitro, CF₃, CN, NR¹¹R¹², NH-CO-R¹³, O-alkyle en C₁-C₄, et
R⁷ étant hydrogène, alkyle en C₁-C₆, phényle, le composé cyclique pouvant encore être substitué par jusqu'à deux radicaux R⁷¹, une amine NR¹¹R¹² ou une amine saturée cyclique à 3 à 7 chaînons, qui peut encore être substituée par un radical alkyle, alkyle en C₁-C₆, ou homopipérazine qui peut encore être substituée par un radical alkyle, alkyle en C₁-C₆, et
les radicaux R¹¹, R¹² et R¹³ dans K, R⁵, R⁶ et R⁷ pouvant prendre indépendamment les uns des autres la même signification que R¹, et
R⁷¹ étant OH, alkyle en C₁-C₆, O-alkyle en C₁-C₄, chlore, brome, iode, fluor, CF₃, nitro ou NH₂, et
R⁸ étant alkyle en C₁-C₆, CF₃, phényle ou alkylphényle en C₁-C₄, le composé cyclique pouvant encore être substitué par jusqu'à deux radicaux R⁸¹, et
R⁸¹ étant OH, alkyle en C₁-C₆, O-C₁-C₄-alkyle, chlore, brome, iode, fluor, CF₃, nitro ou NH₂, et
R⁹ étant hydrogène, alkyle en C₁-C₆, alkylphényle en C₁-C₄, CO₂-alkylphényle en C₁-C₄, CO₂-alkyle en C₁-C₄, SO₂-phényle, COR⁸ ou phényle, les composés cycliques phényliques pouvant encore être substitués par jusqu'à deux radicaux R⁹¹, et
R⁹¹ étant OH, alkyle en C₁-C₆, O-alkyle en C₁-C₄, chlore, brome, iode, fluor, CF₃, nitro ou NH₂,
ainsi que leurs formes tautomères, formes énantiomères et diastéréomères possibles, et leurs promédicaments.

2. Composés de formule I selon la revendication 1,
A étant une chaîne d'hydrocarbures en C₂ qui peut être substituée, et
X¹ représentant O et
R¹ étant hydrogène.

3. Composés de formule I selon l'une quelconque des revendications 1 ou 2,
R⁴ signifiant hydrogène ou D_{0,1}-F¹_{0,1}-G²-G³ avec G³ signifiant hydrogène et
D signifiant O ou NR⁴³, R⁴³ étant hydrogène ou alkyle en C₁-C₃,
et
F¹ signifiant alkyle enC₂-C-₄.

4. Composés sélectionnés parmi le groupe constitué par la 2-(6-nitro-1,3-benzodioxol-5-yl)-5,6-dihydroimidazo[4,5,1-jk][1,4]benzodiazépin-7(4H)-one, la 2-(2,3-dihydro-1,3-benzodioxin-6-yl)-5,6-dihydroimidazo[4,5,1-j,k][1,4]benzodiazépin-7(4H)-one, la 2-(1,3-benzodioxol-5-yl)-5,6-dihydroimidazo[4,5,1-jk][1,4]benzodiazépin-7(4H)-one, la 2-(2,5-diméthoxytétrahydro-3-furanyl)-5,6-dihydroimidazo[4,5,1-jk][1,4]benzodiazépin-7(4H)-one, la 2-(2,3-dihydro-1-benzofuran-5-yl)-5,6-dihydroimidazo[4,5,1-jk][1,4]benzodiazépin-7(4H)-one, la 2-(6-chloro-1,3-benzodioxol-5-yl)-5,6-dihydro-imidazo[4,5,1-jk][1,4]benzodiazépin-7(4H)-one ainsi que leurs formes tautomères, formes énantiomères et diastéréomères possibles, et leurs promédicaments.

5. Médicament contenant outre les supports et adjuvants usuels des composés de formule I selon l'une quelconque des revendications 1 à 4.

6. Utilisation de composés de formule générale I selon l'une quelconque des revendications 1 à 4 en vue de la fabrication de médicaments avec un effet inhibiteur de la PARP.

7. Utilisation de composés de formule I selon la revendication 6 en vue de la fabrication de médicaments pour le traitement de maladies neurodégénératives et de lésions neuronales.

8. Utilisation selon la revendication 6 pour le traitement de telles maladies neurodégénératives et lésions neuronales qui sont déclenchées par l'ischémie, le trauma ou des hémorragies massives.

9. Utilisation selon la revendication 6 pour le traitement de l'accident vasculaire cérébral et du traumatisme crânien.

10. Utilisation selon la revendication 6 pour le traitement de la maladie d'Alzheimer, de la maladie de Parkinson et de la maladie de Huntington.

11. Utilisation de composés de formule I selon la revendication 6 en vue de la fabrication de médicaments pour le traitement ou la prophylaxie de lésions par ischémies.

12. Utilisation de composés de formule I selon la revendication 6 en vue de la fabrication de médicaments pour le traitement d'épilepsies, notamment de crises épileptiques généralisées, comme par exemple le petit mal et de crises toniques-cloniques et de crises partiellement épileptiques, comme l'épilepsie temporale, et de crises complexes partielles.

13. Utilisation de composés de formule I selon la revendication 6 en vue de la fabrication de médicaments pour le traitement de lésions des reins suite à des ischémies rénales, de lésions qui sont provoquées par une thérapie médicamenteuse, comme par exemple pendant la thérapie à la cyclosporine, et pour le traitement pendant et après des transplantations rénales.

14. Utilisation de composés de formule I selon la revendication 6 en vue de la fabrication de médicaments pour le traitement de lésions du coeur après des ischémies cardiaques.

15. Utilisation de composés de formule I selon la revendication 6 en vue de la fabrication de médicaments pour le traitement de microinfarctus, comme par exemple pendant et après le changement de valvules cardiaques, des résections d'anévrismes et des transplantations cardiaques.

16. Utilisation de composés de formule I selon la revendication 6 en vue de la fabrication de médicaments pour le traitement lors d'une revascularisation d'artères coronaires rétrécies de manière critique, comme par exemple lors d'une angioplastie coronarienne transluminale percutanée et d'opérations de pontage ou d'artères périphériques rétrécies de manière critique, notamment d'artères fémorales.

17. Utilisation de composés de formule I selon la revendication 6 en vue de la fabrication de médicaments pour le traitement d'infarctus aigu du myocarde et de lésions pendant et après sa lyse médicamenteuse ou mécanique.

18. Utilisation de composés de formule I selon la revendication 6 en vue de la fabrication de médicaments pour le traitement de tumeurs et de leur capacité à se métastaser.

19. Utilisation de composés de formule I selon la revendication 6 en vue de la fabrication de médicaments pour le traitement de la sepsie, de l'insuffisance d'organes multiples, comme par exemple pendant le choc septique et le syndrome de détresse respiratoire aiguë.

20. Utilisation de composés de formule I selon la revendication 6 en vue de la fabrication de médicaments pour le traitement de maladies immunologiques, comme les inflammations et les maladies rhumatismales, comme par exemple la polyarthrite rhumatoïde.

21. Utilisation de composés de formule I selon la revendication 6 en vue de la fabrication de médicaments pour le traitement du diabète sucré.
